# EUROPEAN PATENT APPLICATION

(11) **EP 1 943 946 A1**
(43) Date of publication of application: **16.07.2008**
(21) Application number: 06811598.9
(22) Date of filing: 11.10.2006
(51) Int. Cl.: A61B 5/05

(54) **BODY COMPOSITION MEASURING DEVICE**

(30) Priority: 31.10.2005 JP 2005316947
(71) Applicant: Omron Healthcare Co., Ltd., Kyoto 615-0084 (JP)
(72) Inventor: ASHIDA, Tameo, Kyoto-shi, Kyoto 615-0084 (JP); KOIKE, Tadashi, Kyoto-shi, Kyoto 615-0084 (JP); SHIGENO, Takashi, Tokyo 107-0062 (JP)
(74) Representative: Wilhelms · Kilian & Partner Patentanwälte
(86) International application number: PCT/JP2006/320291
(87) International publication number: WO 2007/052450

(57) **Abstract**

A body composition measuring apparatus (100A) includes electrodes (131, 133) for a right hand to be contacted with the right hand of a subject and electrodes (132, 134) for a left hand to be contacted with the left hand of the subject in an apparatus body for measuring an impedance of the body of the subject. The apparatus body includes a right hand grip (111) having electrodes (131, 133) for a right hand and extending like a rod, a left hand grip (112) having electrodes (132, 134) for a left hand and extending like a rod, and a base (103) to which right hand grip (111) and left hand grip (112) are attached. Right hand grip (111) is attached to base (103) in a freely rotatable manner, and the extending direction of right hand grip (111) and the direction along which the rotation axis of right hand grip (111) extends are arranged to be different from each other. Thus, a small-sized, thin body composition measuring apparatus capable of being carried along can be obtained.

## Description

### TECHNICAL FIELD

The present invention relates to a body composition measuring apparatus capable of measuring a body composition of a subject by measuring an impedance of the body of the subject.

### BACKGROUND ART

Conventionally, there is known a body composition measuring apparatus that measures a body composition of a subject by measuring an impedance of the body. The body composition measuring apparatus provides useful information for health care, and has become widespread in the home and the like.

The body composition measuring apparatuses are broadly divided into ones using both hands alone as body parts to be contacted with the electrode and ones using both feet as well as both hands as body parts to be contacted with the electrode. The former has a merit over the latter in that the apparatus body can be downsized, and the size of the apparatus body has been reduced to such an extent that allows the apparatus body to be carried along. On the other hand, the latter allows impedance to be measured by each body part, and has a merit over the former in that the body composition can be measured at a higher degree of precision.

For example, as the former, a body composition measuring apparatus is disclosed in Japanese Patent Laying-Open No. 07-51242 (Patent Document 1). In the body composition measuring apparatus disclosed in Patent Document 1, a right hand grip having an electrode for a right hand and a left hand grip having an electrode for a left hand are formed on both right and left ends, respectively, of a main body casing having a display provided thereon. By holding these electrodes with a right hand and a left hand, respectively, a body composition can be measured. At this point, the right hand grip and the left hand grip are disposed spaced from each other such that the spaced distance is approximately equal to the shoulder length of the subject. In this way, consideration is given so that a proper posture for measurement is maintained.

As the latter, for example, a body composition measuring apparatus is disclosed in Japanese Patent Laying-Open No. 2003-220048 (Patent Document 2). In the body composition measuring apparatus disclosed in Patent Document 2, the apparatus body is separated into a first unit and a second unit. These first and second units are wired, and a display, a right hand grip having an electrode for a right hand, and a left hand grip having an electrode for a left hand are formed in the first unit, whereas a right-foot placing portion having an electrode for a right foot and a left-foot placing portion having an electrode for a left foot are formed in the second unit. A subject holds the right hand grip and the left hand grip with the right hand and the left hand, respectively, to keep hold of the first unit, and stands on the second unit with the right foot and the left foot placing on the right-foot placing portion and the left-foot placing portion, respectively. Thus, a body composition can be measured.

In the body composition measuring apparatus disclosed in the foregoing Patent Document 1, which is configured to be able to be carried along, grips having each of the electrodes are formed protruding from both ends of the main body casing to ensure a proper posture for measurement. Therefore, although the apparatus can be carried along, the apparatus is still large, and the apparatus is not necessarily suitable for carrying. To address this shortcoming, the foregoing Patent Document 1 discloses a body composition measuring apparatus in which the grip is attached to the main body casing in a freely rotatable manner so as to be suitable for carrying the apparatus and to render the distance between both electrodes in measurement to be adjustable.

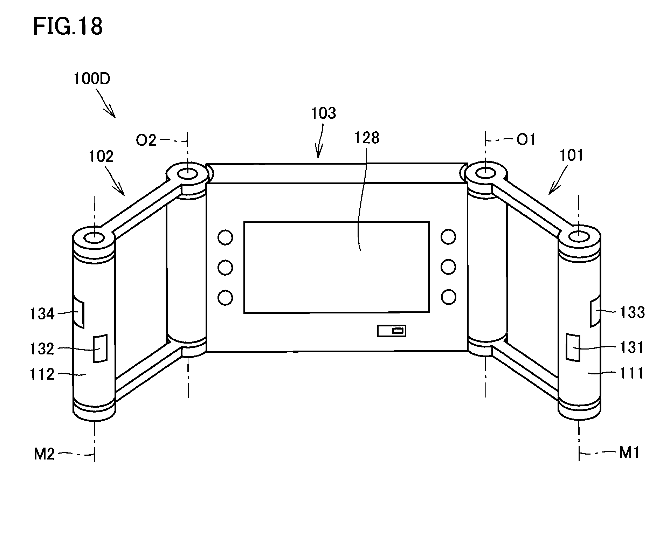

Fig. 18 is a perspective view of a body composition measuring apparatus disclosed in the foregoing Patent Document 1, in which the grip is attached to the main body casing in a freely rotatable manner. As shown in Fig. 18, in this body composition measuring apparatus 100D, a right casing 101 having a right hand grip 111 and a left casing 102 having a left hand grip 112 are attached in a freely rotatable manner to both ends of a main body casing 103 having a display 128. Right hand grip 111 is provided with electrodes 131 and 133 for a right hand, and left hand grip 112 is provided with electrodes 132 and 134 for a left hand. Thus, right casing 101 rotates around a rotation axis O1, and left casing 102 rotates around a rotation axis 02. Therefore, during measurement, the distance between both the electrodes can be optimized by adjusting the distance between both the grips. When the apparatus is not used, the right hand grip and the left hand grip are stored such that they are placed together on top of the main surface of the main body casing having the display provided thereon. For this reason, the body composition measuring apparatus has a more compact outer shape.
Patent Document 1: Japanese Patent Laying-Open No. 07-51242
Patent Document 2: Japanese Patent Laying-Open No. 2003-220048

### DISCLOSURE OF THE INVENTION

### PROBLEMS TO BE SOLVED BY THE INVENTION

However, if the configuration shown in Fig. 18 is employed, right casing 101 and left casing 102 are placed together on top of the main surface of main body casing 103 having display 128 provided thereon when the apparatus is not used, and therefore there arises a problem that main body casing 103 has an increased thickness although it has a reduced length. To stably ensure contact of electrodes with hands, grips 111 and 112 provided in right casing 101 and left casing 102 need to be formed thick enough to fit hands. If these grips 111 and 112 formed thick and main body casing 103 are placed on top one another, the resulting thickness is very large. This is because the extending directions of axis line M1 and axis line M2 of right hand grip 111 and left hand grip 112 each formed in a rod shape are the same as those of rotation axis O1 of right casing 101 and rotation axis 02 of left casing 102, respectively. Therefore, even when the foregoing configuration is employed, the apparatus is still unsuitable for carrying.

The present invention has been made in view of the above problem, and has an object of providing a small-sized, thin body composition measuring apparatus that can be carried along.

### MEANS FOR SOLVING THE PROBLEMS

A body composition measuring apparatus according to a first aspect of the present invention includes, in an apparatus body, a first electrode to be contacted with one hand of a subject, a second electrode to be contacted with the other hand of the subject, and a measurement portion that measures an impedance of a body of the subject using the first electrode and the second electrode. The apparatus body includes a first grip that has the first electrode and extends like a rod, a second grip that has the second electrode and extends like a rod, and a base to which the first grip and the second grip are attached. The first grip is attached to the base in a freely rotatable manner, and an extending direction of the first grip extending like a rod is arranged in a different direction from a direction along which a rotation axis of the first grip extends.

With this configuration, the thickness of the apparatus body is continuously maintained before and after the rotation. This therefore allows the body composition measuring apparatus to be small-sized and thin without being bulky both during measurement and while carrying it.

In the foregoing body composition measuring apparatus according to the first aspect of the present invention, it is preferable that the first grip is rotatable between a first position where measurement by the measurement portion is possible and a second position where measurement by the measurement portion is impossible.

A "position where measurement by the measurement portion is possible" herein refers to a position where the first grip is actually disposed when an impedance is measured, and a "position where measurement by the measurement portion is impossible" refers to a position that is most suitable for carrying the apparatus among positions that the first grip can take when measurement of an impedance is not performed. Hereinafter, a "position where measurement by the measurement portion is possible" will be referred to as a "measurable position" and a state where the first grip is disposed at the position will be referred to as a "measurable state". A "position where measurement by the measurement portion is impossible" will be referred to as a "storage position" and a state where the first grip is disposed at the position will be referred to as a "storage state".

This configuration allows the body composition measuring apparatus to be small-sized and thin without being bulky both in the measurable state and in the storage state.

In the foregoing body composition measuring apparatus according to the first aspect of the present invention, it is preferable that the first position is a position where the first grip is, in a rotatable range thereof, remotest from the second grip, and it is preferable that the second position is a position where the first grip is, in a rotatable range thereof, closest to the second grip. In this case, it is more preferable that when the first grip is at the second position, the extending direction of the first grip is substantially in parallel to an extending direction of the second grip.

This configuration allows the apparatus main body to have a compact and thin outer shape without being bulky when the apparatus body is in the storage state, and also allows the first grip and the second grip to be disposed spaced from each other at a distance approximately equal to the shoulder length of a subject when the apparatus body is in the measurable state. Therefore, a proper posture for measurement can be maintained.

In the foregoing body composition measuring apparatus according to the first aspect of the present invention, it is preferable that a display capable of displaying a body composition based on a measurement result measured by the measurement portion is further included. In this case, it is preferable that the display is exposed in a state where the first grip is at the first position and in a state where the first grip is at the second position.

This configuration allows the display to be visible in either of the measurable state and the storage state. Therefore, a body composition measuring apparatus that is easily handled can be achieved.

In the foregoing body composition measuring apparatus according to the first aspect of the present invention, it is preferable that an engaging mechanism capable of engaging the first grip at the first position and at the second position is further included.

This configuration allows the measurable state where the first grip is at the measurable position and the storage state where the first grip is at the storage position to be maintained by the engaging mechanism. Therefore, a body composition measuring apparatus that can be easily handled can be achieved.

In the foregoing body composition measuring apparatus according to the first aspect of the present invention, it is preferable that a detection portion that detects whether the first grip is at the first position is further included. In this case, it is preferable that when it is determined by the detection portion that the first grip is at the first position, power is configured to be supplied to the measurement portion.

This configuration can eliminate the need to press a power supply button. Therefore, a body composition measuring apparatus that can be easily handled can be achieved.

In the foregoing body composition measuring apparatus according to the first aspect of the present invention, it is preferable that when power is supplied to the measurement portion, measurement by the measurement portion is started immediately or after the lapse of a predetermined time.

With this configuration, a measuring start button need not be provided separately and independently, and therefore the apparatus configuration can be simplified. This also eliminates the need to press a power supply button. Thus, a small-sized body composition measuring apparatus that can be easily handled can be achieved.

In the foregoing body composition measuring apparatus according to the first aspect of the present invention, it is preferable that a detection portion that detects whether the first grip is at the first position is further included. In this case, it is preferable that when it is determined by the detection portion that the first grip is not at the first position, the power supply to the measurement portion is configured to be stopped.

With this configuration, the need to press a power supply button can be eliminated, and forgetting to turn off the power can be prevented. Therefore, a body composition measuring apparatus that can be easily handled can be achieved.

In the foregoing body composition measuring apparatus according to the first aspect of the present invention, it is preferable that the first grip rotates in a plane including an axis line of the first grip extending like a rod and an axis line of the second grip extending like a rod.

With this configuration, the thickness of the apparatus body is continuously maintained before and after the rotation. This therefore allows the body composition measuring apparatus to be small-sized and thin without being bulky both during measurement and while carrying it.

In the foregoing body composition measuring apparatus according to the first aspect of the present invention, the second grip may be attached to the base in a freely rotatable manner. In this case, it is preferable that the extending direction of the second grip extending like a rod is arranged in a different direction from a direction along which a rotation axis of the second grip extends.

With this configuration, the thickness of the apparatus body is continuously maintained before and after the rotation. This therefore allows the body composition measuring apparatus to be small-sized and thin without being bulky both during measurement and while carrying it.

In the foregoing body composition measuring apparatus according to the first aspect of the present invention, it is preferable that a direction along which the rotation axis of the first grip extends and the direction along which the rotation axis of the second grip extends are the same direction. The rotation axis of the first grip and the rotation axis of the second grip may be configured to be overlap with each other on the same straight line.

With this configuration, the thickness of the apparatus body is continuously maintained before and after the rotation. This therefore allows the body composition measuring apparatus to be small-sized and thin without being bulky both during measurement and while carrying it.

A body composition measuring apparatus according to a second aspect of the present invention includes a first electrode to be contacted with one hand of a subject, a second electrode to be contacted with the other hand of the subject, a third electrode to be contacted with one foot of the subject, a fourth electrode to be contacted with the other foot of the subject, and a measurement portion that measures an impedance of the body of the subject by selectively or entirely using the first to fourth electrodes. The first electrode, the second electrode, and the measurement portion are disposed in a first unit that the subject can hold, and the third electrode and the fourth electrode are disposed in a second unit on which the subject can stand. The first unit includes a first grip having the first electrode and extending like a rod, a second grip having the second electrode and extending like a rod, and a base to which the first grip and the second grip are attached. The first grip is attached to the base in a freely rotatable manner, and an extending direction of the first grip extending like a rod is arranged in a different direction from a direction along which a rotation axis of the first grip extends. The second unit has a storage portion that can contain the first unit only when the second grip is at the second position.

With this configuration, the thickness of the first unit is continuously maintained before and after the rotation of the first grip. This therefore allows the first unit to be small-sized and thin without being bulky. Accordingly, the storage portion for the first unit disposed in the second unit can be downsized, and the second unit itself can be downsized. Therefore, a body composition measuring apparatus that is significantly downsized as a whole can be achieved.

In the body composition measuring apparatus according to the second aspect of the present invention, it is preferable that the first unit is wired with the second unit in a freely detachable manner.

This configuration allows selection between a usage mode in which impedance of a body is measured using only the first unit to measure body compositions and a usage mode in which impedance by each body part is measured using the first and second units to measure body compositions at a higher degree of precision. In this case, carrying only the first unit is possible, and containing the first unit in the storage portion of the second unit is also possible. Therefore, a body composition measuring apparatus with improved usabilty can be achieved.

In the body composition measuring apparatus according to the second aspect of the present invention, it is preferable that the display is exposed with the first unit contained in the storage portion.

With this configuration, the display is visible at all times when each of the foregoing usage modes is selected. Therefore, a body composition measuring apparatus with improved usability can be achieved.

### EFFECTS OF THE INVENTION

According to the present invention, a small-sized, thin body composition measuring apparatus capable of being carried along can be achieved.

### BRIEF DESCRIPTION OF THE DRAWINGS

Fig. 1 is a perspective view showing the appearance of a body composition measuring apparatus in the measurable state in Embodiment 1 of the present invention.
Fig. 2 is an exploded perspective view showing an assembling structure of a housing of the body composition measuring apparatus in Embodiment 1 of the present invention.
Fig. 3 is a cross-sectional view of the body composition measuring apparatus in Embodiment 1 of the present invention.
Fig. 4 is a perspective view showing the appearance of the body composition measuring apparatus in the storage state in Embodiment 1 of the present invention.
Fig. 5 is a cross-sectional view of the body composition measuring apparatus in the storage state in Embodiment 1 of the present invention.
Fig. 6 shows functional blocks of the body composition measuring apparatus in Embodiment 1 of the present invention.
Fig. 7 is an enlarged cross-sectional view of an area VII shown in Fig. 5.
Fig. 8A is an enlarged cross-sectional view of an area VIII shown in Fig. 3 immediately before the measurable state.
Fig. 8B is an enlarged cross-sectional view of the area VIII shown in Fig. 3 in the measurable state.
Fig. 9 shows a posture for measurement taken when a subject measures a body composition using the body composition measuring apparatus in Embodiment 1 of the present invention.
Fig. 10 is a flowchart in a case where a body composition is measured using the body composition measuring apparatus in Embodiment 1 of the present invention.
Fig. 11 is a perspective view of a body composition measuring apparatus in Embodiment 2 of the present invention.
Fig. 12 is an exploded perspective view showing an assembling structure of a housing of a body composition measurement unit of the body composition measuring apparatus in Embodiment 2 of the present invention.
Fig. 13 is a perspective view showing a state where the body composition measurement unit is stored in a storage portion of a body weight measurement unit in the body composition measuring apparatus in Embodiment 2 of the present invention.
Fig. 14 shows functional blocks of the body composition measuring apparatus in Embodiment 2 of the present invention.
Fig. 15 shows a posture for measurement taken when a subject measures a body composition using a body composition measuring apparatus 1000 in Embodiment 2 of the present invention.
Fig. 16 is a flowchart in a case where a body composition is measured using the body composition measuring apparatus in Embodiment 2 of the present invention.
Fig. 17 is a top view of a body composition measuring apparatus in Embodiment 3 of the present invention.
Fig. 18 is a perspective view showing one configuration of a conventional body composition measuring apparatus.

### DESCRIPTION OF THE REFERENCE SIGNS

10 Subject, 11 Right hand, 12 Left hand, 13 Right foot, 14 Left foot, 100A, 100C, 100D, 1000 Body composition measuring apparatus, 100B Body composition measurement unit, 101 Right casing, 102 Left casing, 101a, 102a Pivot, 101b, 102b Tooth, 103 Base (main body casing), 111 Right hand grip, 112 Left hand grip, 111a, 112a Annular portion, 111b, 112b Opening, 111c, 112c Protrusion, 113 Cap body, 114 Case body, 114a Storage space, 114b to 114e Opening, 120 Operating portion, 121 Setting button, 122 Measuring start button, 123 Up button, 124 Down button, 128 Display, 128a Display window, 131 to 134 Electrode, 140 Circuit board, 141 Micon, 142 Impedance measurement portion, 143 Body composition calculation portion, 144 Internal memory, 148 Switching unit, 148a Switch, 149 Plate spring, 149a Elastic portion, 149b Abutment, 151 Battery, 150 Connection terminal, 152 High-frequency constant current generating circuit, 153 Voltage measuring circuit, 154 A/D conversion circuit, 185 Body weight measurement portion, 186 Current application electrode switching circuit, 187 Voltage measuring electrode switching circuit, 188 Input switching circuit, 200 Body weight measurement unit, 210 Pedestal, 211 Storage portion, 213 Right-foot placing portion, 214 Left-foot placing portion, 221 to 224 Electrode, 231 Power supply button, 232 Connecting cord, 240 Cord winding unit

### BEST MODES FOR CARRYING OUT THE INVENTION

Preferred embodiments of the present invention will be described in detail below with reference to the accompanying drawings. Regarding the embodiments to be described below, it should be noted that the same components are denoted by the same reference numerals in the drawings, and the description thereof is not repeated.

### (Embodiment 1)

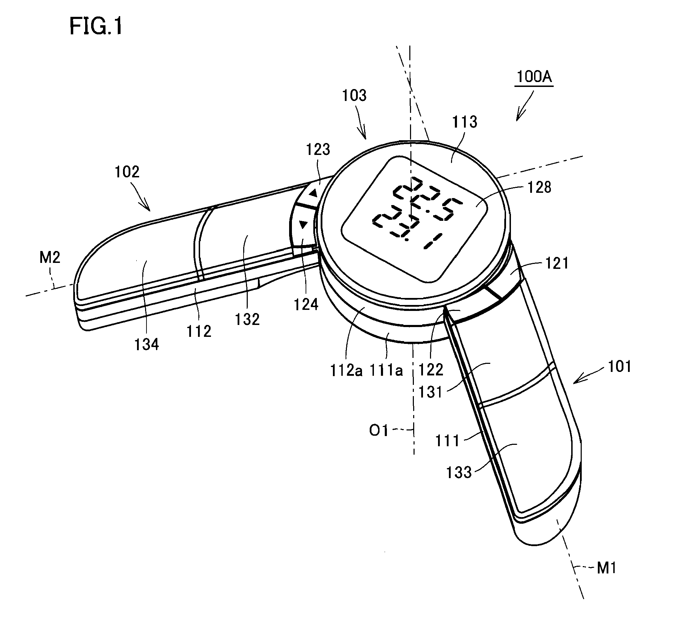

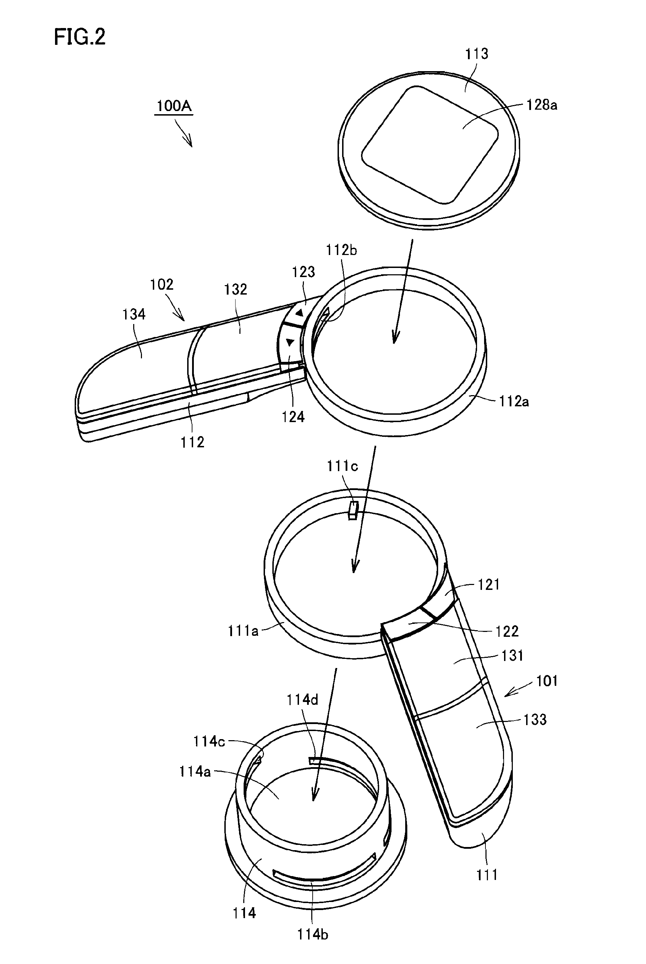

Fig. 1 a perspective view showing the appearance of a body composition measuring apparatus in the measurable state in Embodiment 1 of the present invention. Fig. 2 is an exploded perspective view showing an assembling structure of a housing of a body composition measuring apparatus shown in Fig. 1. With reference to these figures, an appearance configuration in the measurable state of the body composition measuring apparatus and the assembling structure of the housing in the present embodiment are first described.

As shown in Fig. 1, a body composition measuring apparatus 100A in the present embodiment includes a right hand grip 111 to be held with a right hand and a left hand grip 112 to be held with a left hand. Each of grips 111 and 112 is formed in a rod shape so that it can be held with a hand. In body composition measuring apparatus 100A in the present embodiment, right hand grip 111 corresponds to a first grip, and the left hand grip 112 corresponds to a second grip.

As shown in Figs. 1 and 2, a casing constituting the outer shell of an apparatus body of body composition measuring apparatus 100A includes a right casing 101, a left casing 102, and a cap body 113 and a case body 114 as a base 103.

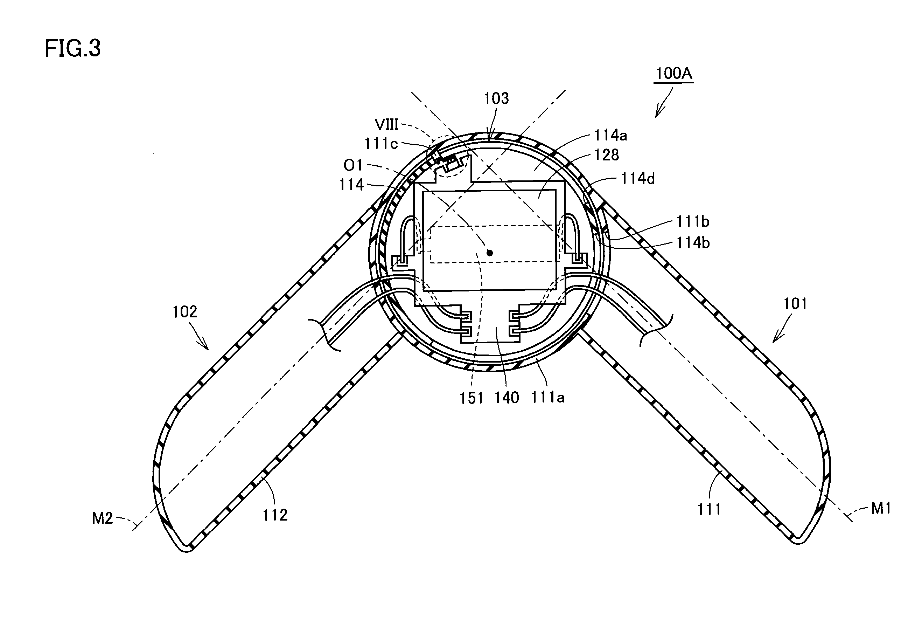

Right casing 101 has right hand grip 111 formed of a rod-like portion extending in one direction, and an annular portion 111a extending continuously from one end of right hand grip 111 to be formed like a ring. Right hand grip 111 has a hollow inside. To communicate with the hollow, an opening 111b (see Fig. 3) is provided in a portion following right hand grip 111 of annular portion 111a. A protrusion 111c protrudes inward at a predetermined position on the inner peripheral surface of annular portion 111a of right casing 101. Note that the axis line of right hand grip 111 extending like a rod is denoted by M1 in Fig. 1. The extending direction of this axis line M1 corresponds to that of right hand grip 111.

Left casing 102 has left hand grip 112 formed of a rod-like portion extending in one direction, and an annular portion 112a extending continuously from one end of left hand grip 112 to be formed like a ring. Left hand grip 112 has a hollow inside. To communicate with the hollow, an opening 112b is provided in a portion following left hand grip 112 of annular portion 112a. Note that the axis line of left hand grip 112 extending like a rod is denoted by M2 in Fig. 1. The extending direction of this axis line M2 corresponds to that of left hand grip 112.

Case body 114, formed of a closed-end cylindrical member having an opened top surface, has a storage space 114a inside. A plurality of openings 114b, 114c, and 114d are provided at predetermined positions of the circumferential wall of case body 114. Among these openings, opening 114b is provided at such a position as to face opening 111b provided in right casing 101 after assembly, and opening 114c at such a position as to face opening 112b provided in left casing 102 after assembly. Opening 114d is provided at a position corresponding to protrusion 111c provided in annular portion 111a of right casing 101, and thus protrusion 111c is moved in opening 114d after assembly. Note that a flange is formed outward at the lower end of the outer peripheral surface of case body 114.

Cap body 113 is formed of a plate member that can close the top opening of case body 114, and has, at a predetermined position of the top surface thereof, a display window 128a constituting a display surface of a display 128.

Annular portion 111a of right casing 101 and annular portion 112a of left casing 102 are inserted, in this order, into the circumferential wall of case body 114. Cap body 113 is attached to case body 114 with annular portion 111a of right casing 101 and annular portion 112a of left casing 102 inserted thereto so as to close storage space 114a. At this point, annular portion 111a of right casing 101 and annular portion 112a of left casing 102 are sandwiched by the flange of case body 114 and the outer periphery of cap body 113 on the outer peripheral surface of case body 114. Left casing 102 is fixed to case body 114 and cap body 113, each by a fixing mechanism (not shown), so as to be unable to be moved relative to each other. As a result, right casing 101 is assembled rotatably relative to left casing 102, case body 114, and cap body 113 integrated by the above fixing mechanism. Note that the rotation axis of right casing 101 (i.e., rotation axis of right hand grip 111) is denoted by O1 in Fig. 1.

As shown in Figs. 1 and 2, electrodes 131 and 133 are provided at predetermined positions on the outer surface of right hand grip 111 of right casing 101. In these electrodes 131 and 133, electrode 131 positioned on the side of annular portion 111a is an electrode for measuring voltage during impedance measurement, and electrode 133 positioned on the opposite side to the annular portion 111a side is an electrode for applying an electric current during impedance measurement. These electrodes 131 and 133 are first electrodes to be contacted with the inside of the right hand of the subject.

Electrodes 132 and 134 are provided at predetermined positions on the outer surface of left hand grip 112 of left casing 102. In these electrodes 132 and 134, electrode 132 positioned on the side of annular portion 112a is an electrode for measuring voltage during impedance measurement, and electrode 134 positioned on the opposite side to the annular portion 112a side is an electrode for applying an electric current during impedance measurement. These electrodes 132 and 134 are second electrodes to be contacted with the inside of the left hand of the subject.

As described above, cap body 113 is provided with display window 128a, and formed in this portion is display 128. Display 128 is a display unit for displaying a body composition and the like measured by this body composition measuring apparatus 100A. In body composition measuring apparatus 100A in the present embodiment, a display surface of display 128 is arranged substantially in parallel to a plane including axis line M1 of right hand grip 111 and axis line M2 of left hand grip 112. Note that a liquid crystal display (LCD) or the like is utilized as display 128.

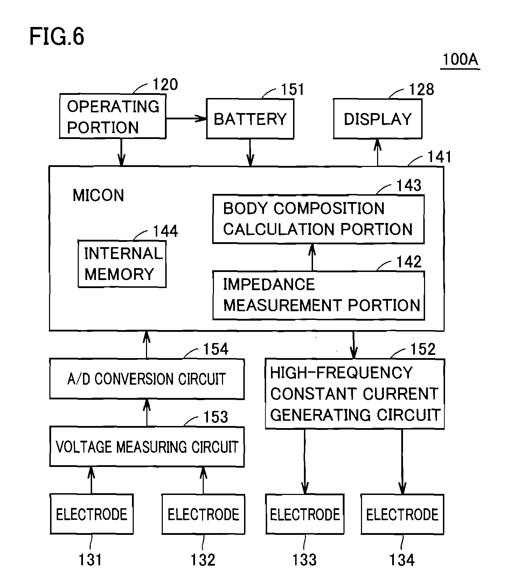

Provided in a portion adjacent to display 128 of right casing 101 are a setting button 121 for performing various settings and a measuring start button 122 for providing an instruction for starting measurement. Provided in a portion adjacent to display 128 of left casing 102 are an Up button 123 and a Down button 124 for selecting a value to be inputted, e.g., at the time of setting personal data. These setting button 121, measuring start button 122, Up button 123 and Down button 124 correspond to an operating portion 120 for receiving operations of the subject (see Fig. 6).

Fig. 3 is a cross-sectional view of the body composition measuring apparatus in the present embodiment. With reference to this figure, an internal structure of the body composition measuring apparatus in the present embodiment will be described next.

As described above, in body composition measuring apparatus 100A in the present embodiment, hollows are provided inside right hand grip 111 of right casing 101 and inside left hand grip 112 of left casing 102, and storage space 114a is provided inside case body 114. Various parts constituting body composition measuring apparatus 100A are contained in these hollows and storage space 114a.

Specifically, as shown in Fig. 3, a circuit board 140 is disposed in storage space 114a of case body 114. Various circuits to be described later are formed by mounting various electronic parts on this circuit board 140. In addition, display 128 is provided on this circuit board 140. A battery 151 is contained in storage space 114a in a portion positioned below circuit board 140. Battery 151 is electrically connected to circuit board 140 with a lead wire.

Lead wires are connected to electrodes 131 and 133 provided on right hand grip 111 of right casing 101 by soldering and the like. These lead wires are drawn through opening 111b provided in right casing 101 and opening 114b provided in case body 114 into storage space 114a of case body 114 to be electrically connected to circuit board 140. Lead wires are also connected to electrodes 132 and 134 provided on left hand grip 112 of left casing 102 by soldering and the like. These lead wires are drawn through opening 112b (see Fig. 2) provided in left casing 102 and opening 114c (see Fig. 2) provided in case body 114 into storage space 114a of case body 114 to be electrically connected to circuit board 140.

In body composition measuring apparatus 100A in the present embodiment, as described above, right hand grip 111 of right casing 101 rotates relatively to left hand grip 112 of left casing 102. The rotational movements are possible only in a plane including axis line M1 of right hand grip 111 and axis line M2 of left hand grip 112, and the rotatable range is determined by the length in the circumferential direction of opening 114d provided in the circumferential wall of case body 114. In body composition measuring apparatus 100A of the present embodiment, the rotation angle of right hand grip 111 is approximately 90° by adjusting the length of opening 114d. However, the rotation angle is not particularly limited, and is preferably an angle within a range of 60° to 180°, and more preferably, an angle within a range of 90° to 120°.

By employing the structure described above, body composition measuring apparatus 100A in the present embodiment may be in the measurable state where right hand grip 111 is remotest from left hand grip 112 in the rotatable range, and may also be in the storage state where right hand grip 111 is closest to left hand grip 112 in the rotatable range. In the following, a case of body composition measuring apparatus 100A in the storage state will be described in detail.

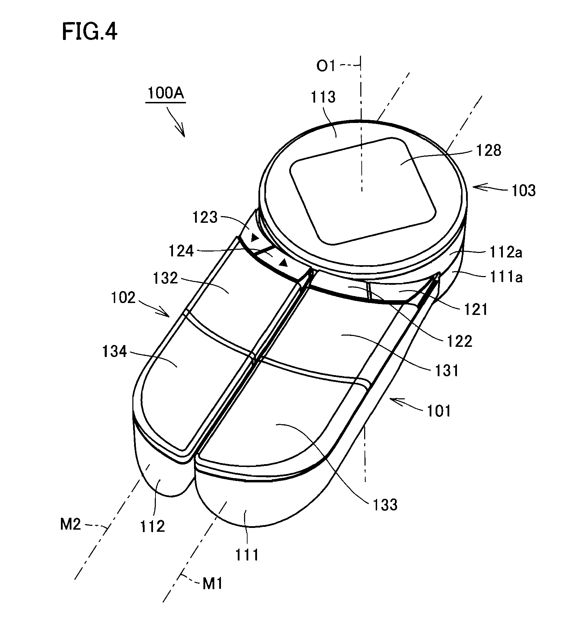

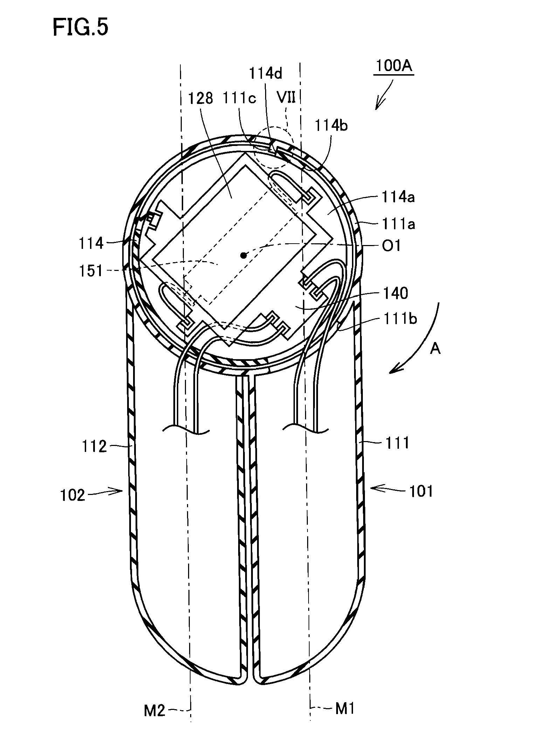

Fig. 4 is a perspective view showing the appearance of the body composition measuring apparatus in the storage state in the present embodiment. Fig. 5 is a cross-sectional view of the body composition measuring apparatus in the storage state in the present embodiment.

As shown in Figs. 4 and 5, in the storage state, right casing 101 and left casing 102 are disposed such that the extending direction of axis line M1 of right hand grip 111 of right casing 101 and the extending direction of axis line M2 of left hand grip 112 of left casing 102 are substantially in parallel to each other. Specifically, as shown in Fig. 5, right hand grip 111 is rotated in the arrow A direction in the figure, thereby arranging both extending the directions of grips 111 and 112 substantially in parallel to each other. In this case, display 128 is maintained in a state of being exposed. Since cap body 113 having display 128 provided thereon is fixed to left casing 102 as described above, the vertical direction of the display surface of display 128 is inclined with respect to the extending directions of right hand grip 111 and left hand grip 112 in a plane including axis line M1 of right hand grip 111 and axis line M2 of left hand grip 112.

In body composition measuring apparatus 100A in the present embodiment, as shown in Figs. 1 and 4, right casing 101 including right hand grip 111 is rotatably attached to base 103 constituted by case body 114 and cap body 113 and is disposed so that the extending direction of axis line M1 of right hand grip 111 is different from that of rotation axis O1 of right hand grip 111. With this configuration, the thickness of the body of the apparatus is continuously maintained before and after the rotation, allowing the body composition measuring apparatus to be compact and thin without being bulky both during measurement and while carrying it. Display 128 is exposed both in the measurable state and in the storage state, and therefore is visible in both the states, allowing the body composition measuring apparatus to be easily handled.

In the measurable state, right hand grip 111 and left hand grip 112 are disposed spaced from each other at a distance approximately equal to the shoulder length of the subject, and therefore a proper posture for measurement is maintained. This enables a compact shape of the apparatus in the storage state as compared with that in the measurable state. Therefore, a small-sized body composition measuring apparatus suitable for carrying can be achieved.

Fig. 6 shows functional blocks of the body composition measuring apparatus in the present embodiment. With reference to this figure, the functional blocks of the body composition measuring apparatus in the present embodiment will be described next.

As shown in Fig. 6, body composition measuring apparatus 100A in the present embodiment includes, in addition to electrodes 131 to 134, display 128, operating portion 120, and battery 151, a microcomputer (micon) 141 for performing control over entire body composition measuring apparatus 100A and performing processes such as various operations, a high-frequency constant current generating circuit 152 that generates a high-frequency constant current of a predetermined frequency, a voltage measuring circuit 153 that measures voltage information obtained from electrodes 131 and 132 for voltage measurement, and an analog/digital (A/D) conversion circuit 154 for converting voltage information obtained from voltage measuring circuit 153 from analog signals to digital signals. Micon 141 includes an impedance measurement portion 142 that measures an impedance of a body from the voltage information in digital signals, a body composition calculation portion 143 that calculates a body composition by computing the obtained impedance, and an internal memory 144 for storing various control programs and the like.

It should be noted that examples of the body composition that can be measured with body composition measuring apparatus 100A of the present embodiment include an amount of body fat, a lean body mass, a muscle mass, a bone mass, a percent of body fat, a muscle percentage, and a visceral fat level. All of these body compositions are calculated from personal data such as impedance values of a body obtained in impedance measurement portion 142 described above and height, body weight, age, and gender of the subject stored in the internal memory using a known method by body composition calculation portion 143.

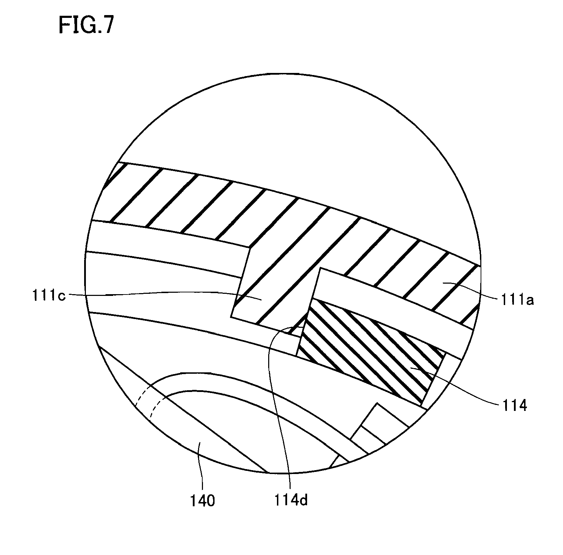

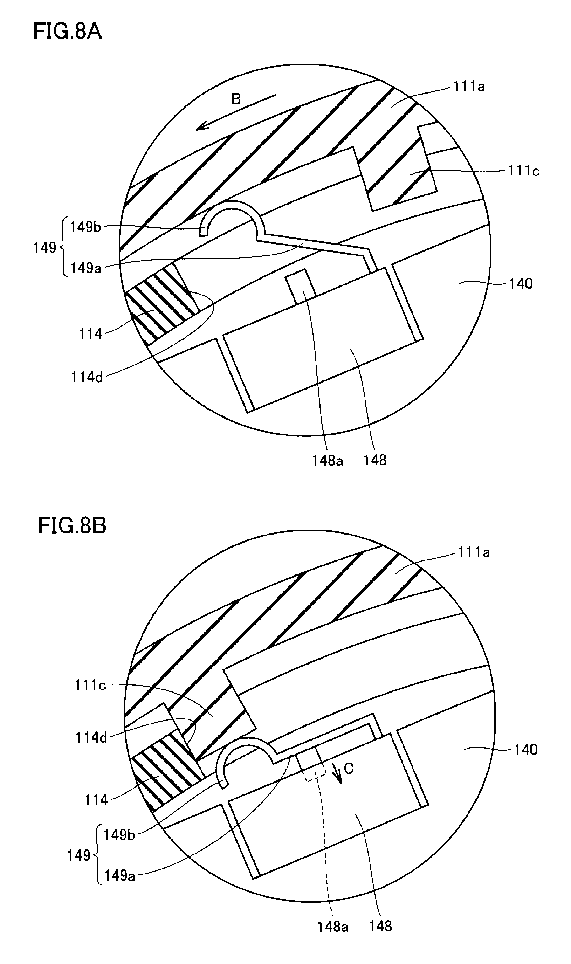

Fig. 7 is an enlarged cross-sectional view of an area VII shown in Fig. 5, and Figs. 8A and 8B are enlarged cross-sectional views of an area VIII shown in Fig. 3. Note that Fig. 8A shows the area immediately before the measurable state is established, and Fig. 8B shows the area when the measurable state has been established. With reference to these figures, On/Off operations of a power supply involved in rotational movements of the right casing will be described below.

Body composition measuring apparatus 100A in the present embodiment is provided with a detection portion that detects whether right casing 101 having right hand grip 111 provided thereon is at a measurable position. This detection portion serves as a switch for supplying power to micon 141 when right hand grip 111 is at the measurable position. As shown in Figs 3 and 5, a switching unit 148 (see Figs. 8A and 8B) as the detection portion is attached to a predetermined position of circuit board 140.

As shown in Figs. 8A and 8B, a plate spring 149 is attached to switching unit 148. Switching unit 148 has a switch 148a, and power is supplied in conjunction with the On/Off operations of this switch 148a. Plate spring 149 includes an elastic portion 149a extending linearly and an abutment 149b positioned at the tip of elastic portion 149a and having a curved shape, and switch 148a is disposed at a position corresponding to elastic portion 149a. Plate spring 149 is attached to switching unit 148 so as to exert biasing force toward the outside of circuit board 140. Switch 148a is turned on only when protrusion 111c provided in annular portion 111a of right casing 101 is in contact with abutment 149b of plate spring 149.

Specifically, as shown in Fig. 7, when the apparatus body is in the storage state, protrusion 111c provided in annular portion 111a of right casing 101 is positioned in one end of opening 114d on the circumferential wall of case body 114 such that protrusion 111c is present at a position apart from plate spring 149. Accordingly, in this state, body composition measuring apparatus 100A is in the Off state where power is not supplied.

However, as shown in Fig. 8A, when right casing 101 is rotated in the arrow B direction in the figure by the subject, protrusion 111c moves in opening 114d toward an end at which switching unit 148 is provided. Then, as shown in Fig. 8B, when right hand grip 111 reaches the measurable position, protrusion 111c is brought into contact with abutment 149b of plate spring 149. Against the elastic force that elastic portion 149a has, abutment 149b of plate spring 149 is pushed in toward switching unit 148. This causes switch 148a to be pressed down in the arrow C direction in the figure by elastic portion 149a of plate spring 149 to be in the On state. With this operation, power supply to micon 141 is started.

Note that when right hand grip 111 at the measurable position is rotated toward the storage position, pressing force to plate spring 149 is released as protrusion 111c moves, causing switch 148a to be in the Off state. With this operation, power supply to micon 141 is stopped.

With this configuration, the need to press the power supply button can be eliminated, and forgetting to turn off the power can be prevented. The need to press the measuring start button can also be eliminated. Accordingly, a body composition measuring apparatus that can be easily handled can be achieved. Further, since the power supply button and the measuring start button need not be provided separately and independently, the apparatus configuration can be simplified. Thus, the body composition measuring apparatus can be configured to have a small size.

Note that if a concave having a shape corresponding to abutment 149b of plate spring 149 is provided at the tip of protrusion 11c, abutment 149b of plate spring 149 is fitted into and engaged with the concave. In a state of right hand grip 111 disposed at the measurable position, right hand grip 111 can thus be engaged at the position. Further, if, in a state of right hand grip 111 disposed at the storage position, a concave is provided on the inner peripheral surface of right casing 101 at a position corresponding to a portion where abutment 149b of plate spring 149 is positioned, abutment 149b of plate spring 149 is fitted into and engaged with this concave. This enables right hand grip 111 to be engaged at the position in the state of right hand grip 111 placed at the storage position. This configuration enables the measurable state where right hand grip 111 is at the measurable position and the storage state where right hand grip 111 is at the storage position to be maintained. Therefore, a body composition measuring apparatus that can be easily handled can be obtained.

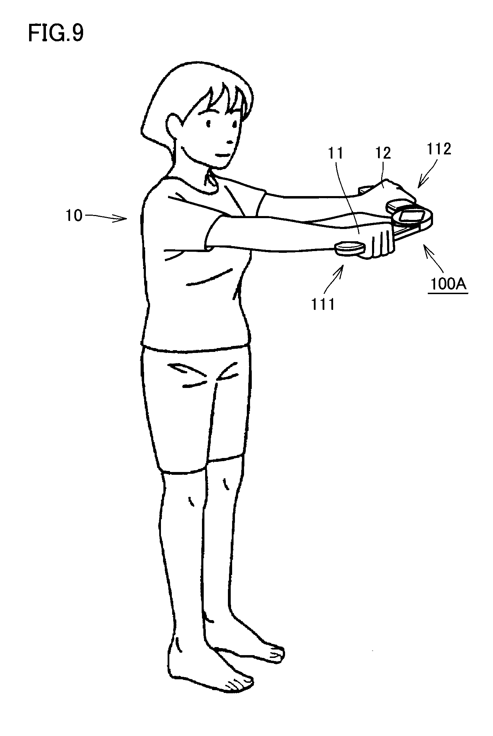

Fig. 9 shows a posture for measurement taken when the subject measures a body composition using the body composition measuring apparatus in the present embodiment. As shown in Fig. 9, a subject 10 holds right hand grip 111 and left hand grip 112 of body composition measuring apparatus 100A with a right hand 11 and with a left hand 12, respectively, while taking an upright posture. At this point, the elbows of both arms are extended, and both the arms are maintained at a height approximately equal to that of the shoulder so that body composition measuring apparatus 100A is positioned in front of the body, and the arms and the trunk are approximately perpendicular to each other.

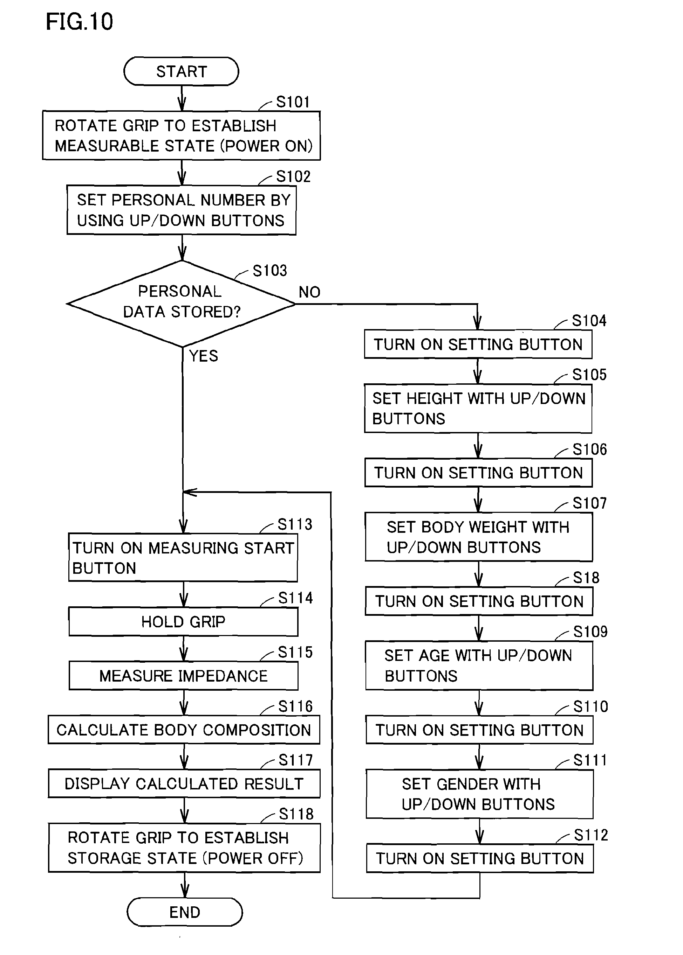

Fig. 10 is a flowchart in the case where a body composition is measured using the body composition measuring apparatus in the present embodiment. With reference to this figure, a flow at the time of measuring a body composition will be described below.

As shown in Fig. 10, when a body composition is measured, first in step S101, right hand grip 111 is rotated with respect to left hand grip 112 to establish the measurable state. At this point, switch 148a is turned on to supply power to micon 141. Next, in step S102, a personal number with which personal data is recorded is set by using Up button 123 and Down button 124. At this point, it is determined in step S103 whether the personal data is stored with the personal number. If the personal data is not stored, then the process proceeds to step S104, and setting button 121 is pressed down. Thereafter, in steps S105 to S112, Up button 123, Down button 124, and setting button 121 are operated to set personal data such as height, body weight, age and gender, in succession.

If personal data is stored in step S103 or if setting of personal data is completed in steps S 104 to S 112, then the process proceeds to step S 113, and measuring start button 122 is pressed down. Thereafter, in step S 114, both grips 111 and 112 are held in the posture as shown in Fig. 9. In step S 115, an impedance is measured; in step S 116, a body composition is calculated; and in step S 117, the calculated result of the body composition is displayed on display 128. When measurement is completed, in step S 118, right hand grip 111 is rotated with respect to left hand grip 112 to establish the storage state. At this point, switch 148a is turned off to stop power supply to micon 141.

### (Embodiment 2)

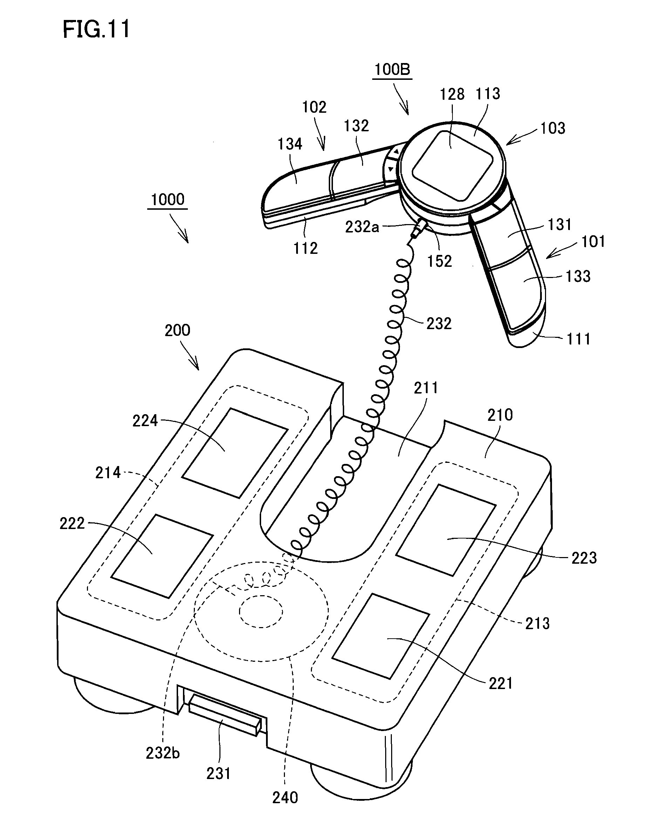

Fig. 11 is a perspective view of a body composition measuring apparatus in Embodiment 2 of the present invention. With reference to this figure, an appearance configuration of the body composition measuring apparatus in the present embodiment will first be described.

As shown in Fig. 11, a body composition measuring apparatus 1000 in the present embodiment includes a body composition measurement unit 100B as a first unit and a body weight measurement unit 200 as a second unit. Body composition measurement unit 100B has substantially the same configuration as that of body composition measuring apparatus 100A in Embodiment 1 described above. Specifically, body composition measurement unit 100B includes right casing 101 having right hand grip 111 to be held with a right hand, left casing 102 having left hand grip 112 to be held with a left hand, and base 103 having display 128 provided thereon. Electrodes 131 and 133 as first electrodes are provided at predetermined positions on the outer surface of right casing 101, and electrodes 132 and 134 as second electrodes are provided at predetermined positions on the outer surface of left casing 102.

Body weight measurement unit 200 has a pedestal 210 on which the subject is to stand. Provided on the top surface of this pedestal 210 are a right-foot placing portion 213 on which the right foot of the subject is to be placed and a left-foot placing portion 214 on which the left foot of the subject is to be placed. Provided on the heel side of right-foot placing portion 213 is an electrode 221 for measuring voltage in impedance measurement, and provided on the toe side of right-foot placing portion 213 is an electrode 223 for applying an electric current during impedance measurement. These electrodes 221 and 223 correspond to third electrodes to be contacted with the sole of the right foot of the subject. Provided on the heel side of left-foot placing portion 214 is an electrode 222 for measuring voltage in impedance measurement, and provided on the toe side of left-foot placing portion 214 is an electrode 224 for applying an electric current in impedance measurement. These electrodes 222 and 224 correspond to fourth electrodes to be contacted with the sole of the left foot of the subject.

Provided at a predetermined position on pedestal 210 of body weight measurement unit 200 is a storage portion 211 for containing body composition measurement unit 100B, and a connecting cord 232 is drawn out from a predetermined position of this storage portion 211. Connecting cord 232 has a first end 232b fixed to a cord winding unit 240 provided inside pedestal 210 and a second end 232a provided with a plug so as to be connected to a connection terminal 150 provided in body composition measurement unit 100B in a freely detachable manner. Note that a power supply button 231 is provided at a predetermined position in pedestal 210 of body weight measurement unit 200.

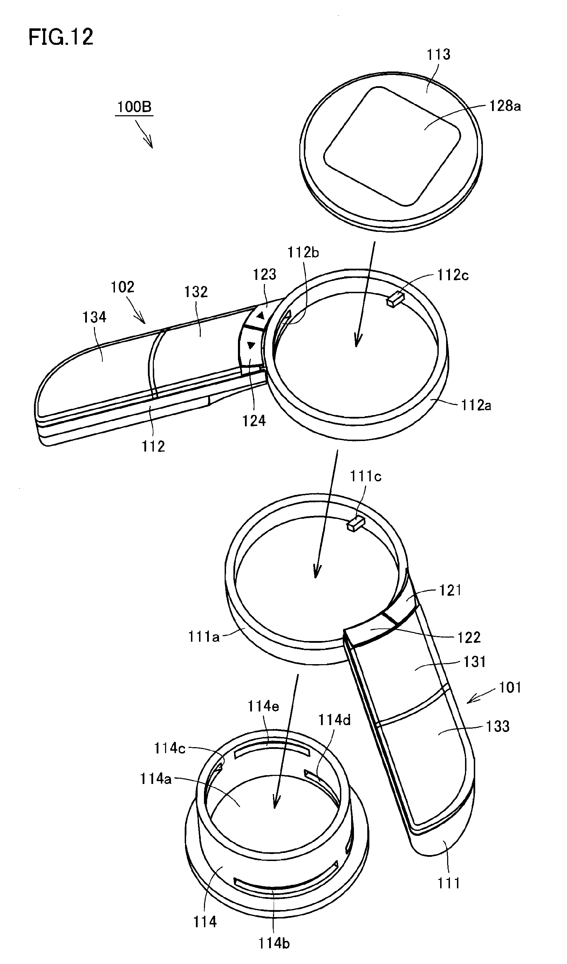

Fig. 12 is an exploded perspective view showing an assembling structure of a housing of the body composition measurement unit of the body composition measuring apparatus in the present embodiment. With reference to Fig. 12, the structure of the housing of the body composition measurement unit in the present embodiment will be described next.

In body composition measuring apparatus 1000 in the present embodiment also, body composition measurement unit 100B includes right casing 101, left casing 102, and cap body 113 and case body 114 as base 103, just as in body composition measuring apparatus 100A of Embodiment 1 described above.

Right casing 101 has right hand grip 111 formed of a rod-like portion extending in one direction, and annular portion 111a extending continuously from one end of right hand grip 111 to be formed like a ring. Protrusion 111c protrudes inward at a predetermined position on the inner peripheral surface of annular portion 111a of right casing 101. On the other hand, left casing 102 has left hand grip 112 formed of a rod-like portion extending in one direction, and annular portion 112a extending continuously from one end of left hand grip 112 to be formed like a ring. Protrusion 112c protrudes inward at a predetermined position on the inner peripheral surface of annular portion 112a of left casing 102.

Case body 114, formed of a closed-end cylindrical member having an opened top surface, has storage space 114a inside. The plurality of openings 114b, 114c, 114d, and 114e are provided at predetermined positions on the circumferential wall of case body 114. Among these openings, opening 114d is provided at a position corresponding to protrusion 111c provided in annular portion 111a of right casing 101, and thus protrusion 111c is moved in opening 114d after assembly. Opening 114e is provided at a position corresponding to protrusion 112c provided in annular portion 112a of left casing 102, and thus protrusion 112c is moved in opening 114e after assembly.

Annular portion 111a of right casing 101 and annular portion 112a of left casing 102 are inserted, in this order, into the circumferential wall of case body 114. Cap body 113 is attached to case body 114 with annular portion 111a of right casing 101 and annular portion 112a of left casing 102 inserted thereto so as to close storage space 114a. At this point, annular portion 111a of right casing 101 and annular portion 112a of left casing 102 are sandwiched by the flange of case body 114 and the outer periphery of cap body 113 on the outer peripheral surface of case body 114. Case body 114 and cap body 113 are fixed so as to be unable to be moved relative to each other by a fixing mechanism (not shown). Therefore, right casing 101 and left casing 102 are assembled rotatably relative to base 103 constituted by case body 114 and cap body 113. Note that this causes the rotation axis of right casing 101 and the rotation axis of left casing 102 to be disposed on the same straight line.

In body composition measuring apparatus 100B in the present embodiment, as described above, right hand grip 111 of right casing 101 and left hand grip 112 of left casing 102 each rotate relatively to base 103. The rotational movements are possible only in a plane including the axis line of right hand grip 111 and the axis line of left hand grip 112, and the rotatable range is determined by the lengths in the circumferential direction of openings 114d and 114e provided in the circumferential wall of case body 114. In body composition measurement unit 100B of the present embodiment, the rotation angles of right hand grip 111 and left hand grip 112 are each approximately 45° by adjusting the lengths of openings 114d and 114e. However, the rotation angles are not particularly limited, and are preferably angles within a range of 30° to 90°, and more preferably, angles within a range of 45° to 60°.

By employing the structure described above, body composition measurement unit 100B in the present embodiment can be in the measurable state where right hand grip 111 and left hand grip 112 are remotest from each other in the rotatable range and in the storage state where right hand grip 111 and left hand grip 112 are closest to each other in the rotatable range. With this configuration, the thickness of the apparatus body is continuously maintained before and after the rotation, allowing the body composition measuring apparatus to be compact and thin without being bulky both during measurement and while carrying it. Display 128 is exposed both in the measurable state and in the storage state, and therefore is visible in both the states, allowing the body composition measurement unit to be excellent in operability. In the measurable state, right hand grip 111 and left hand grip 112 are disposed apart from each other at a distance approximately equal to the shoulder length of the subject, and therefore a proper posture for measurement is maintained. This enables a compact shape of the apparatus in the storage state as compared with that in the measurable state.

Regarding body composition measuring apparatus 100A in Embodiment 1 described above, in its storage state, the vertical direction of the display surface of display 128 is inclined with respect to the extending directions of right hand grip 111 and left hand grip 112 in a plane including axis line M1 of right hand grip 111 and axis line M2 of left hand grip 112. However, in body composition measurement unit 100B in the present embodiment, since cap body 113 having display 128 provided thereon is not fixed to both right casing 101 and left casing 102, the vertical direction of the display surface of display 128 can be in the same direction as the extending direction of right hand grip 111 and left hand grip 112 in a plane including axis line M1 of right hand grip 111 and axis line M2 of left hand grip 112 by adjusting positions of forming protrusions 111c and 112c, and openings 114d and 114e.

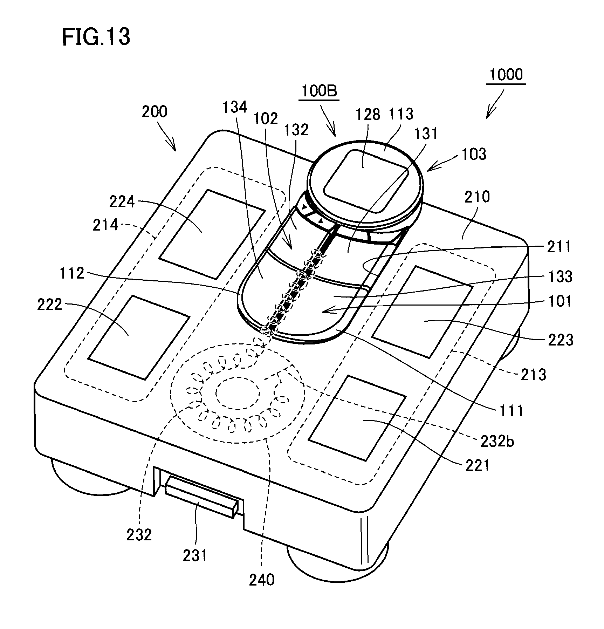

In body composition measuring apparatus 1000 in the present embodiment, body composition measurement unit 100B is configured to be able to be stored in storage portion 211 of body weight measurement unit 200 only when body composition measurement unit 100B is in the storage state. Fig. 13 is a perspective view showing a state where the body composition measurement unit is contained in the storage portion of the body weight measurement unit in the body composition measuring apparatus in the present embodiment.

As shown in Fig. 13, with body composition measurement unit 100B contained in storage portion 211 of body weight measurement unit 200, one end 232, fixed to cord winding unit 240, of connecting cord 232 that connects body composition measurement unit 100B with body weight measurement unit 200 is wound by this cord winding unit 240. As a result, most of connecting cord 232 is drawn and contained in body weight measurement unit 200. The top surface of body composition measurement unit 100B in the storage state is exposed on the top surface of body weight measurement unit 200, and display 128 of body composition measurement unit 100B is also exposed. In addition, in this state, body composition measuring apparatus 1000 may be configured to operate in a mode of body weight measurement only unless power supply button 231 is turned on and body composition measurement unit 100B is taken out of storage portion 211. This enables a body composition measuring apparatus excellent in convenience to be achieved.

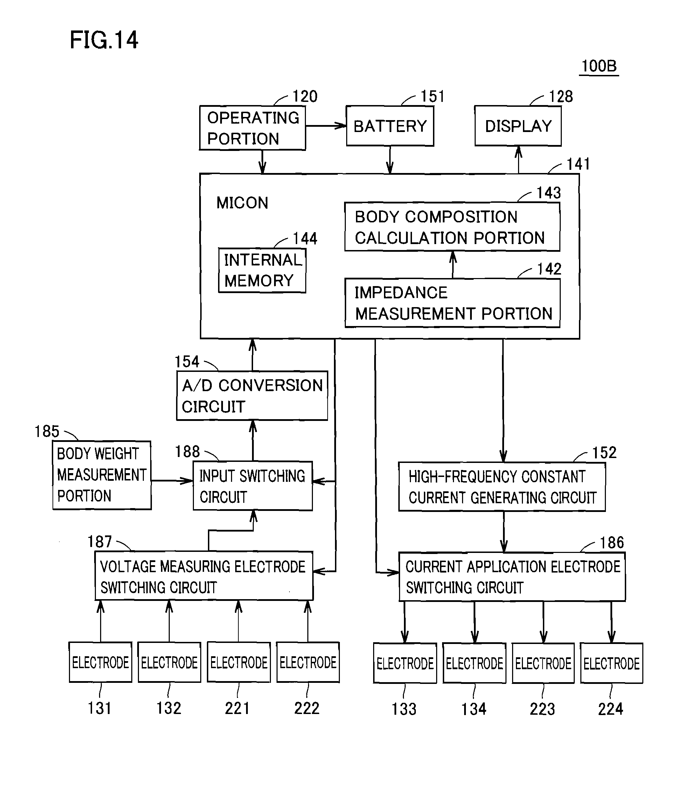

Fig. 14 shows functional blocks of the body composition measuring apparatus in the present embodiment. With reference to this figure, the functional blocks of the body composition measuring apparatus in the present embodiment will be described next.

As shown in Fig. 14, body composition measuring apparatus 1000 in the present embodiment includes, in addition to the plurality of electrodes 131 to 134 and 221 to 224, display 128, and operating portion 120, which have been described above, a micon 141 for performing control over entire body composition measuring apparatus 1000 and performing processes such as various operations, a high-frequency constant current generating circuit 152 that generates a high-frequency constant current of a predetermined frequency, a body weight measurement portion 185 for measuring a body weight, a current application electrode switching circuit 186 for switching electrodes 133, 134, 223, and 224 for applying an electric current, a voltage measuring electrode switching circuit 187 for switching electrodes 131, 132, 221, and 222 for voltage measurement, an input switching circuit 188 for switching input to either voltage information obtained from voltage measuring electrode switching circuit 187 or body weight information obtained from body weight measurement portion 185, an A/D conversion circuit 154 for converting voltage information and body weight information obtained from input switching circuit 188 from analog signals to digital signals, and a battery 151 for supplying power to micon 141. Micon 141 includes an impedance measurement portion 142 that measures an impedance of a body from voltage information in digital signals, a body composition calculation portion 143 that calculates a body composition by computing the obtained impedance, and an internal memory 144 for storing various control programs and the like.

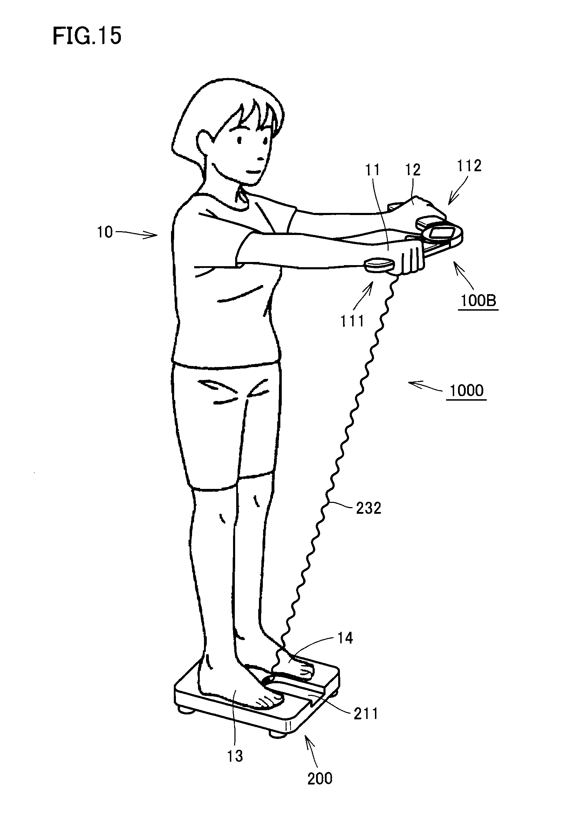

Fig. 15 shows a posture for measurement taken when a subject measures a body composition using body composition measuring apparatus 1000 in the present embodiment. As shown in Fig. 15, subject 10 places a right foot 13 and a left foot 14 on right-foot placing portion 213 and left-foot placing portion 214 of body weight measurement unit 200, respectively, and holds right hand grip 111 and left hand grip 112 of body composition measurement unit 100B with right hand 11 and left hand 12, respectively, while taking the upright posture. At this point, the elbows of both the arms are extended, and both the arms are maintained at a height approximately equal to that of the shoulder so that body composition measurement unit 100B is positioned in front of the body, and the arms and the trunk are approximately perpendicular to each other.

In body composition measuring apparatus 1000 in the present embodiment, an electrode to be used is switched using current application electrode switching circuit 186 and voltage measuring electrode switching circuit 187. This allows impedance by each body part to be measured. A "body part" herein refers to one of a plurality of parts into which the whole body except the head portion of the subject to be measured is divided, and represents one of the torso and the limbs or one of parts into which they are further divided. As the dividing method, for example, the whole body is generally divided into five body parts, namely, a left arm part, a right arm part, a left leg part, a right leg part, and a trunk part.

Note that examples of body compositions that can be measured in body composition measuring apparatus 1000 in the present embodiment include an amount of body fat, a lean body mass, a muscle mass, a bone mass, a percent of body fat, a muscle percentage, and a visceral fat level of the whole body or of each body part. All these body compositions are calculated from impedance values of the body obtained in impedance measurement portion 142 and personal data such as height, age, and gender of the subject recorded in the internal memory using a known method by body composition calculation portion 143.

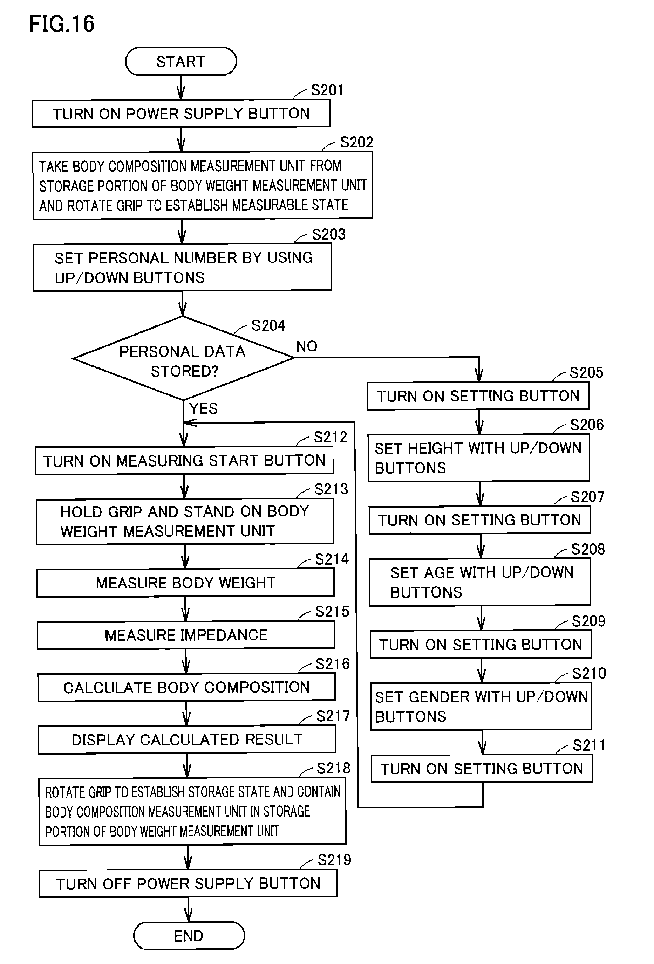

Fig. 16 is a flowchart in the case where a body composition is measured using the body composition measuring apparatus in the present embodiment. With reference to this figure, a flow at the time of measuring a body composition will be described below.

As shown in Fig. 16, when a body composition is measured, first, in step S201, power supply button 231 provided in body weight measurement unit 200 is pressed down. Then, in step S202, body composition measurement unit 100B is taken from storage portion 211 of body weight measurement unit 200, right hand grip 111 and left hand grip 112 are rotated to establish the measurable state. Next, in step S203, a personal number with which personal data is recorded is set by using Up button 123 and Down button 124. At this point, it is determined in step S204 whether the personal data is stored with the personal number. If the personal data is not stored, then the process proceeds to step S205 and setting button 121 is pressed down. Thereafter, in steps S206 to S211, Up button 123, Down button 124, and setting button 121 are operated to set personal data such as height, age and gender, in succession.

If personal data is stored in step S204 or when setting of personal data is completed in steps S206 to S211, then the process proceeds to step S212, and measuring start button 122 is pressed down. Thereafter, in step S213, the subject stands on body weight measurement unit 200 and holds both grips 111 and 112 in the posture as shown in Fig. 16. In step S214, the body weight is measured; next in step S215, an impedance is measured; in step S216, a body composition is calculated; and in step S217, the calculated result of the body composition is displayed on display 128. When measurement is completed, in step S218, right hand grip 111 and left hand grip 112 are rotated to establish the storage state, and body composition measurement unit 100B is contained in storage portion 211 of body weight measurement unit 200. Then, in step S219, power supply button 231 is pressed down.

Body composition measuring apparatus 1000 configured as described above allows selection between usage modes, as appropriate. In one usage mode, body composition measurement unit 100B is removed from body weight measurement unit 200 by removing connecting cord 232, and an impedance of the body is measured using the removed body composition measurement unit 100B to measure a body composition. In the other usage mode, body composition measurement unit 100B and body weight measurement unit 200 are connected with connecting cord 232, and impedance by each body part is measured using these units to measure body compositions at a higher degree of precision. As a result, only body composition measurement unit 100B can be carried along, while body composition measurement unit 100B can be contained in storage portion 211 of body weight measurement unit 200. Therefore, a body composition measuring apparatus with improved usability can be achieved. By employing the configuration described above, the outer shape of the apparatus as a whole can be significantly downsized. Note that the display is visible at all times when either of the foregoing usage modes is selected. Therefore, a body composition measuring apparatus with improved usability can be achieved.

### (Embodiment 3)

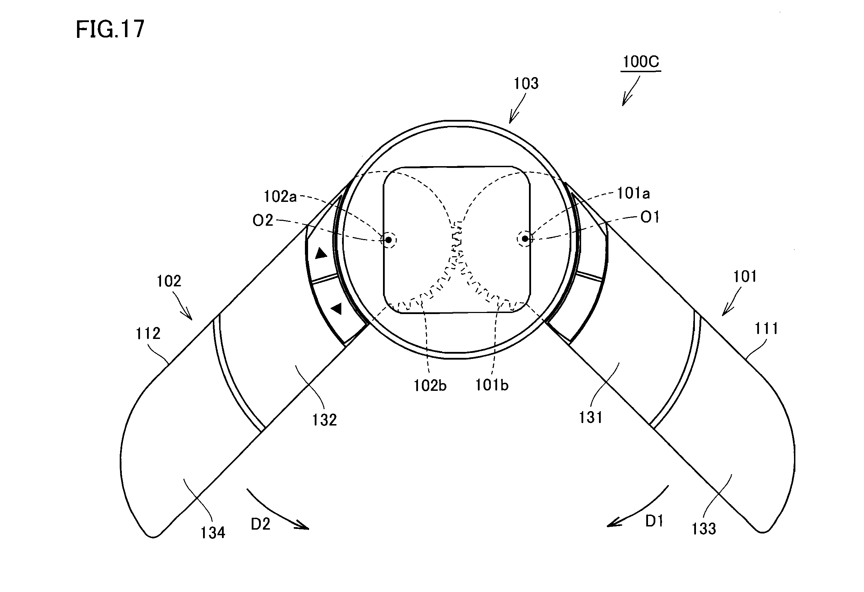

Fig. 17 is a top view of a body composition measuring apparatus in Embodiment 3 of the present invention. In a body composition measuring apparatus 100C in the present embodiment, just as in body composition measurement unit 100B in Embodiment 2 described above, both right casing 101 and left casing 102 rotate with respect to base 103. However, right casing 101 and left casing 102 are rotatably supported by pivots 101a and 102a, respectively, that are disposed at different positions on base 103. That is, rotation axis O1 of right hand grip 111 and rotation axis 02 of left hand grip 112 are disposed at different positions. However, rotation axis O1 and rotation axis 02 are disposed in parallel.

Teeth 101b are provided in an end contained in base 103 of right casing 101, whereas teeth 102b are provided in an end contained in base 103 of left casing 102. These teeth 101b and teeth 102b are mesh with each other and configured such that rotating one grip causes rotation of the other grip. For example, when right hand grip 111 is rotated in the arrow D 1 direction in the figure, left hand grip 112 is rotated in the arrow D2 direction in the figure. Thus, if right hand grip 111 and left hand grip 112 are interlocked using some link mechanism, more improved operability can be achieved.

In Embodiments 1 to 3 described above, descriptions have been given by way of example of a case where the display surface of display 128 is substantially in parallel to a plane including axis line M1 of right hand grip 111 and axis line M2 of left hand grip 112. These surfaces do not have to be parallel, but may be non-parallel. In particular, it is more preferable in terms of visibility that when the subject takes the posture for measurement as shown in Figs. 9 and 15, display 128 is disposed such that the display surface of display 128 faces the subject. However, such a configuration as in Embodiment 2 described above is based on the assumption that the body weight is measured in a state of body composition measurement unit 100B contained in the storage portion of body weight measurement unit 200. Therefore, it is necessary to properly adjust the angle of inclination to prevent the visibility from being impaired even in this case.

Also, in Embodiments 1 to 3 described above, descriptions have been given by way of example of a case where body composition measuring apparatus 100A, body composition measurement unit 100B, and body composition measuring apparatus 100C are not provided with power supply buttons. However, as a matter of course, they may be separately provided with power supply buttons.

Also, in Embodiments 1 to 3 described above, descriptions have been given by way of example of a case where, when right hand grip 111 and/or left hand grip 112 are/is rotated to establish the measurable state, power is automatically supplied. In addition to this case, when power is supplied to micon 141, measurement by micon 141 may start immediately or after the lapse of a predetermined time.

Also, in Embodiment 2 described above, descriptions have been given by way of example of a case where body composition measurement unit 100B and body weight measurement unit 200 are wired in a freely detachable manner. However, the connecting cord does not have to be freely detachable, but may be undetachable.

It is to be understood that the foregoing embodiments disclosed herein are illustrative in all respects, and are not restrictive. It will be clear that technical scope of the present invention is defined by the following claims, and includes all changes and modifications within the scope and meaning of the description of claims and equivalents thereof.

## Claims

**1.** A body composition measuring apparatus comprising in an apparatus body:
a first electrode to be contacted with one hand of a subject;
a second electrode to be contacted with the other hand of the subject; and
a measurement portion that measures an impedance of a body of the subject using said first electrode and said second electrode, wherein
said apparatus body includes:
a first grip having said first electrode and extending like a rod;
a second grip having said second electrode and extending like a rod; and
a base to which said first grip and said second grip are attached,
said first grip is attached to said base in a freely rotatable manner, and
an extending direction of said first grip extending like a rod is arranged in a different direction from a direction along which a rotation axis of said first grip extends.

**2.** The body composition measuring apparatus according to claim 1, wherein said first grip is rotatable between a first position where measurement by said measurement portion is possible and a second position where measurement by said measurement portion is impossible.

**3.** The body composition measuring apparatus according to claim 2, wherein said first position is a position where said first grip is, in a rotatable range thereof, remotest from said second grip, and
said second position is a position where said first grip is, in a rotatable range thereof, closest to said second grip.

**4.** The body composition measuring apparatus according to claim 2, wherein when said first grip is at said second position, the extending direction of said first grip extending like a rod and an extending direction of said second grip extending like a rod are the same direction.

**5.** The body composition measuring apparatus according to claim 2, further comprising a display capable of displaying a body composition based on a measurement result measured by said measurement portion, wherein
said display is exposed in a state of said first grip being at said first position and in a state of said first grip being at said second position.

**6.** The body composition measuring apparatus according to claim 2, further comprising an engaging mechanism capable of engaging said first grip at said first position and at said second position.

**7.** The body composition measuring apparatus according to claim 2, further comprising a detection portion that detects whether said first grip is at said first position, wherein
when it is detected by said detection portion that said first grip is at said first position, power is configured to be supplied to said measurement portion.

**8.** The body composition measuring apparatus according to claim 7, wherein when power is supplied to said measurement portion, measurement by said measurement portion is started immediately or after the lapse of a predetermined time.

**9.** The body composition measuring apparatus according to claim 2, further comprising a detection portion that detects whether said first grip is at said first position, wherein
when it is detected by said detection portion that said first grip is not at said first position, power supply to said measurement portion is configured to be stopped.

**10.** The body composition measuring apparatus according to claim 1, wherein said first grip rotates in a plane including an axis line of said first grip extending like a rod and an axis line of said second grip extending like a rod.

**11.** The body composition measuring apparatus according to claim 1, wherein said second grip is attached to said base in a freely rotatable manner, and
the extending direction of said second grip extending like a rod is arranged in a different direction from a direction along which a rotation axis of said second grip extends.

**12.** The body composition measuring apparatus according to claim 11, wherein a direction along which the rotation axis of said first grip extends and a direction along which the rotation axis of said second grip extends are the same direction.

**13.** The body composition measuring apparatus according to claim 11, wherein the rotation axis of said first grip and the rotation axis of said second grip are disposed to overlap with each other on the same straight line.

**14.** A body composition measuring apparatus comprising:
a first electrode to be contacted with one hand of a subject;
a second electrode to be contacted with the other hand of the subject;
a third electrode to be contacted with one foot of the subject;
a fourth electrode to be contacted with the other foot of the subject; and
a measurement portion that measures an impedance of a body of the subject by selectively or entirely using said first to fourth electrodes, wherein
said first electrode, said second electrode, and said measurement portion are disposed in a first unit that the subject can hold,
said third electrode and said fourth electrode are disposed in a second unit on which the subject can stand,
said first unit includes a first grip having said first electrode, a second grip having said second electrode, and a base to which said first grip and said second grip are attached,
said first grip is attached to said base in a freely rotatable manner,
an extending direction of said first grip extending like a rod is arranged in a different direction from a direction along which a rotation axis of said first grip extends, and
said second unit has a storage portion capable of containing said first unit only when said first grip is at said second position.

**15.** The body composition measuring apparatus according to claim 14, wherein said first unit is wired with said second unit in a freely detachable manner.

**16.** The body composition measuring apparatus according to claim 14, wherein said first unit further includes a display capable of displaying a body composition based on a measurement result measured by said measurement portion, and
said display is exposed with said first unit contained in said storage portion.

**1.** (Amended) A body composition measuring apparatus comprising in an apparatus body:
a first electrode to be contacted with one hand of a subject;
a second electrode to be contacted with the other hand of the subject; and
a measurement portion that measures an impedance of a body of the subject using said first electrode and said second electrode, wherein
said apparatus body includes:
a first grip having said first electrode and extending like a rod;
a second grip having said second electrode and extending like a rod; and
a base to which said first grip and said second grip are attached,
said first grip is attached to said base in a freely rotatable manner to be rotatable between a first position where measurement by said measurement portion is possible and a second position where measurement by said measurement portion is impossible,
an extending direction of said first grip extending like a rod is arranged in a different direction from a direction along which a rotation axis of said first grip extends,
said apparatus body further includes a detection portion that detects whether said first grip is at said first position, and
when it is determined by said detection portion that said first grip is at said first position, power is configured to be supplied to said measurement portion.

**2.** (Canceled)

**3.** (Amended) The body composition measuring apparatus according to claim 1, wherein
said first position is a position where said first grip is, in a rotatable range thereof, remotest from said second grip, and
said second position is a position where said first grip is, in a rotatable range thereof, closest to said second grip.

**4.** (Amended) The body composition measuring apparatus according to claim 1, wherein when said first grip is at said second position, the extending direction of said first grip extending like a rod and an extending direction of said second grip extending like a rod are the same direction.

**5.** (Amended) The body composition measuring apparatus according to claim 1, further comprising a display capable of displaying a body composition based on a measurement result measured by said measurement portion, wherein
said display is exposed in a state of said first grip being at said first position and in a state of said first grip being at said second position.

**6.** (Amended) The body composition measuring apparatus according to claim 1, further comprising an engaging mechanism capable of engaging said first grip at said first position and at said second position.

**7.** (Canceled)

**8.** (Amended) The body composition measuring apparatus according to claim 1, wherein when power is supplied to said measurement portion, measurement by said measurement portion is started immediately or after the lapse of a predetermined time.

**9.** (Amended) The body composition measuring apparatus according to claim 1, wherein when it is detected by said detection portion that said first grip is not at said first position, power supply to said measurement portion is configured to be stopped.

**10.** The body composition measuring apparatus according to claim 1, wherein said first grip rotates in a plane including an axis line of said first grip extending like a rod and an axis line of said second grip extending like a rod.

**11.** The body composition measuring apparatus according to claim 1, wherein said second grip is attached to said base in a freely rotatable manner, and
the extending direction of said second grip extending like a rod is arranged in a different direction from a direction along which a rotation axis of said second grip extends.

**12.** The body composition measuring apparatus according to claim 11, wherein a direction along which the rotation axis of said first grip extends and a direction along which the rotation axis of said second grip extends are the same direction.

**13.** The body composition measuring apparatus according to claim 11, wherein the rotation axis of said first grip and the rotation axis of said second grip are disposed to overlap with each other on the same straight line.

**14.** A body composition measuring apparatus comprising:
a first electrode to be contacted with one hand of a subject;
a second electrode to be contacted with the other hand of the subject;
a third electrode to be contacted with one foot of the subject;
a fourth electrode to be contacted with the other foot of the subject; and
a measurement portion that measures an impedance of a body of the subject by selectively or entirely using said first to fourth electrodes, wherein
said first electrode, said second electrode, and said measurement portion are disposed in a first unit that the subject can hold,
said third electrode and said fourth electrode are disposed in a second unit on which the subject can stand,
said first unit includes a first grip having said first electrode, a second grip having said second electrode, and a base to which said first grip and said second grip are attached,
said first grip is attached to said base in a freely rotatable manner,
an extending direction of said first grip extending like a rod is arranged in a different direction from a direction along which a rotation axis of said first grip extends, and
said second unit has a storage portion capable of containing said first unit only when said first grip is at said second position.

**15.** The body composition measuring apparatus according to claim 14, wherein said first unit is wired with said second unit in a freely detachable manner.

**16.** The body composition measuring apparatus according to claim 14, wherein said first unit further includes a display capable of displaying a body composition based on a measurement result measured by said measurement portion, and
said display is exposed with said first unit contained in said storage portion.
